# EUROPÄISCHE PATENTSCHRIFT

(11) **EP 0 968 167 B1**
(45) Veröffentlichungstag und Bekanntmachung des Hinweises auf die Patenterteilung: **25.09.2002**
(21) Anmeldenummer: 98906938.0
(22) Anmeldetag: 09.02.1998
(51) Int. Cl.: C07C 51/50, C07C 51/43, C07C 55/14

(54) **Verfahren zur Herstellung von Dicarbonsäurekristallisaten**
Process for the preparation of dicarboxylic acid crystals
Procédé de préparation de cristaux d'acides dicarboxyliques

(30) Priorität: 12.02.1997 DE 19705329
(43) Veröffentlichungstag der Anmeldung: 05.01.2000
(73) Patentinhaber: BASF AKTIENGESELLSCHAFT, 67056 Ludwigshafen (DE)
(72) Erfinder: RAULS, Matthias, D-67117 Limburgerhof (DE); BAUMANN, Dieter, D-67227 Frankenthal (DE); WISTUBA, Hermann, D-68259 Mannheim (DE); OTTO, Bernhard, D-67117 Limburgerhof (DE)
(74) Vertreter: Isenbruck, Günter, Dr.
(86) Internationale Anmeldenummer: EP9800703
(87) Internationale Veröffentlichungsnummer: WO98035929

(56) Entgegenhaltungen:
- DE-A- 1 618 796
- US-A- 3 330 665
- US-A- 5 296 639
- DAVEY R J ET AL: "STRUCTURAL AND KINETIC FEATURES OF CRYSTAL GROWTH INHIBITION: ADIPIC ACID GROWING IN THE PRESENCE OF N-ALKANOIC ACIDS" JOURNAL OF THE CHEMICAL SOCIETY. FARADAY TRANSACTIONS, Bd. 88, Nr. 23, 7.Dezember 1992, Seiten 3461-3466, XP000323755 in der Anmeldung erwähnt
- DATABASE WPI Section Ch, Week 8541 Derwent Publications Ltd., London, GB; Class A96, AN 85-253810 XP002068698 & JP 60 169 436 A (NIPPON SHOKUBAI KAGAKU KOGYO CO LTD)

## Beschreibung

Die vorliegende Erfindung betrifft ein Verfahren zur Herstellung von Dicarbonsäurekristallisaten, insbesondere von riesel- und lagerfähigen Dicarbonsäurekristallisaten. Weiterhin betrifft die Erfindung die Verwendung von einem oder mehreren anionischen Polyelektrolyten bei der Herstellung von Dicarbonsäurekristallisaten.

Kristallisierte Dicarbonsäuren, und unter diesen insbesondere die Adipinsäure, sind ein weitverbreiteter Grundstoff zur chemischen Synthese, beispielsweise zur Herstellung von Polymeren, insbesondere von Polyamiden. Um eine leichte Verarbeitbarkeit und Handhabung zu gewährleisten, werden die Dicarbonsäuren in der Regel zu Kristallpulvern (Kristallisaten) kristallisiert. Hierbei sollen die Kristallisate jedoch keine zu geringe mittlere Kristallgrößenverteilung aufweisen, um beispielsweise Staubbildung bei der Handhabung zu verringern oder zu vermeiden.

Solche Kristallisate weisen jedoch die Eigenschaft auf, bei längerer Lagerung im Haufwerk zu größeren Kristalliten zu verbacken. Größere Transport- und Lagergebinde wie Big Bags oder Silos lassen sich daher häufig nur unter erheblichem mechanischen Aufwand zur Auflockerung verbackenen Kristallisats entleeren. Dieser Umstand verursacht beispielsweise bei der Anwendung von Adipinsäure einen unerwünschten zusätzlichen Zeit- und Kostenaufwand.

Adipinsäure kristallisiert aus reinen Lösungen gewöhnlich in Form von dünnen Blättchen, die eine große Berührungsfläche aufweisen und damit aufgrund attraktiver Wechselwirkungen zwischen den einzelnen Berührungsflächen gute Haftung zwischen benachbarten Kristallen ermöglichen. Adipinsäurekristallisate sind beispielsweise in R.J. Davey et al. in *J. Chem. Soc. Faraday Trans*., **88** (23), 3461-3466 (1992) beschrieben.

In der oben genannten Literatur wird auch dargelegt, daß die Oberfläche reiner Adipinsäurekristalle im wesentlichen durch die in {100}-Richtung orientierten kristallographischen Flächen bestimmt wird, deren physikalische Eigenschaften sich aus den hier liegenden hydrophilen Carboxylgruppen ergeben. Werden zwei solcher {100}-Flächen miteinander in Kontakt gebracht, so können sie durch Ausbildung von Wasserstoffbrückenbindungen sofort schwach aneinander haften bleiben. In Gegenwart geringster Wassermengen kann dann bei längerer Lagerung eine stabilere kristalline Brücke zwischen den Kristallen aufgebaut werden. Die Ausbildung solcher kristallinen Brücken ist für das oben beschriebene Verbacken des Kristallisats verantwortlich.

Ein weiterer Nachteil von Adipinsäurekristallisaten ist darauf zurückzuführen, daß die ausgebildeten Kristallplättchen sehr dünn sind. Dünne Kristallplättchen brechen sehr leicht im Laufe des Herstellungs- oder Verarbeitungsprozesses und erzeugen hierdurch einen in der Regel unerwünschten Feinanteil. Die damit einhergehende Verbreiterung der Kristallgrößenverteilung wird zum einen häufig empirisch mit einer Verschlechterung des Rieselverhaltens in Verbindung gebracht, zum anderen führt der Feinanteil zur Staubbildung bei der Verarbeitung, wodurch Produktverluste verursacht werden können und gegebenenfalls aufwendige Maßnahmen zur Gewährleistung der Arbeitssicherheit durchzuführen sind.

Im Stand der Technik werden eine Reihe von physikalischen und chemischen Verfahren beschrieben, die eine Unterbindung des Verbackungsvorgangs erlauben. So wird beispielsweise bei der Lagerung von Adipinsäure in einem Produktsilo der Silo kontinuierlich mit kleinen Mengen eines trockenen Gases durchströmt. Da mit diesem Gasstrom stets vorhandene Feuchtigkeitsspuren weitgehend abgeführt werden, bleibt die Ausbildung interkristalliner Brücken im wesentlichen aus, und eine Verbackung kann so weitgehend verhindert werden. Diese Methode weist aber den Nachteil auf, daß sie nur schwer auf Transportbehälter angewandt werden kann, insbesondere nicht auf Big Bags.

Eine weitere Methode, um die starke interkristalline Haftung zu unterbinden, ist die Belegung der Kristalle mit hydrophobierenden Mitteln. So beschreibt beispielsweise die DE-A 1,618,796 mehrere Möglichkeiten, wie durch Aufbringen von Monocarbonsäuren auf Adipinsäurekristalle deren Oberfläche hydrophobiert und die Ausbildung von interkristallinen Brücken dadurch verhindert werden kann. Nachteilig an diesem Verfahren ist, daß der Adipinsäure zwischen 20 und 100 ppm an Fettsäuren zugesetzt werden müssen, die im Produkt verbleiben und es damit für Anwendungen mit hohen Reinheitsanforderungen ungeeignet machen. Außerdem erfordert diese Methode einen zusätzlichen Verfahrensschritt bei der Herstellung der Adipinsäure.

Die US-A 5,296,639 beschreibt ein Verfahren zur Reinigung von Adipinsäuren während der Kristallisation, bei dem die Kristallmorphologie derart modifiziert wird, daß sich die Aufnahme von Verunreinigungen während der Kristallisation verringert. Zu diesem Zweck werden beispielsweise Capronsäure oder ausgewählte Tenside wie beispielsweise Natriumdodecylsulfat, Natriumdodecylsulfonat oder Natriumdodecylbenzolsulfonat zugegeben. Nachteilig wirkt sich bei diesem Verfahren aus, daß die Additive typischerweise in Konzentrationen von mehr als 100 ppm bis zu 3% zugesetzt werden müssen, um den gewünschten Effekt zu erzielen. Hierdurch wird das Produkt in der Regel unzulässig kontaminiert. Zusätzlich besteht beim Einsatz von Tensiden noch der Nachteil, daß sie im Falle einer Aufpegelung durch interne Rückführung des Lösungsmittels (in der Regel Wasser) in Anlagen zur Schaumbildung führen, so daß eine Anwendung im konkreten technischen Prozeß in der Regel erschwert wird oder sogar überhaupt nicht möglich ist.

Die DE-OS 2 303 627 betrifft die Kristallisation von Persalzen, wobei der Persalzlösung vor der Kristallisation ein oder mehrere wasserlösliche Polyelektrolyte zugegeben werden. Die Druckschrift erwähnt zwar die Zugabe von Polyacrylaten, auf die Bedeutung des Molekulargewichts wird hierbei jedoch nicht eingegangen. Weiterhin ist der Druckschrift nicht zu entnehmen, daß bereits eine geringe Menge an Polyelektrolyt ausreicht, um zu Dicarbonsäurekristalliten mit ausreichender Größe und Stabilität bei gleichzeitig geringem Restfeuchtigkeitsgehalt und ausgezeichneter, auf einer besonderen Isometrie der Kristalle beruhenden Rieselfähigkeit zu gelangen.

Aufgabe der Erfindung war es daher, ein Verfahren zur Herstellung von Dicarbonsäurekristallisaten und damit solche Dicarbonsäurekristallisate zur Verfügung zu stellen, welche die oben genannten Nachteile des Standes der Technik nicht aufweisen. Insbesondere war es Aufgabe der Erfindung, mittels eines geeigneten Verfahrens Dicarbonsäurekristallisate zur Verfügung zu stellen, die ein gutes Rieselverhalten zeigen, und die auch bei längerer Lagerung sowohl in Produktsilos wie auch in Trarisportbehältern, wie beispielsweise Big Bags, ihre Rieselfähigkeit nicht verlieren. Ebenfalls war es Aufgabe der Erfindung, Dicarbonsäurekristallisate zur Verfügung zu stellen, die weder während des Herstellungsprozesses noch bei der Handhabung, beim Transport oder vor oder während der Verarbeitung eine hohe Tendenz zur Bildung von Feinanteilen zeigen. Eine weitere Aufgabe der Erfindung war es, mittels eines geeigneten Verfahrens Dicarbonsäurekristallisate zur Verfügung zu stellen, die eine hohe Produktreinheit besitzen und weitgehend frei von Verunreinigungen durch Belegmittel oder Kristallisationshilfinittel sind.

Es wurde nun gefunden, daß die oben genannten Aufgaben dadurch gelöst werden, daß eine Lösung einer Dicarbonsäure unter Zugabe mindestens eines anionischen Polyelektrolyten mit einer Molmasse von wenigstens 2.000, in einer Menge von 0,01 bis 200 ppm, als Kristallisationshilfsmittel einer Kristallisation unterzogen wird.

Gegenstand der Erfindung ist damit ein Verfahren zur Herstellung von Dicarbonsäurekristallisaten aus einer mindestens eine organische Dicarbonsäure enthaltenden Lösung, wobei der Lösung vor oder während der Kristallisa-tion mindestens ein anionischer Polyelektrolyt mit einer Molmasse von wenigstens 2.000, in einer Menge von 0,01 bis 200 ppm, zugegeben wird.

Alle Zahlenangaben im Rahmen dieses Textes beziehen sich auf das Gewicht der jeweils bezeichneten Komponenten, insofern nicht ausdrücklich etwas anderes angegeben ist. Der Begriff "Polyelektrolyt", wie er der Einfachheit halber im Rahmen dieses Textes benutzt wird, bezieht sich stets auf das gesamte eingesetzte Kristallisationshilfsmittel, d.h. sowohl auf einen einzelnen Polyelektrolyten als auch auf ein Gemisch aus mindestens zwei Polyelektrolyten.

Das nach diesem Verfahren erhältliche Dicarbonsäurekristallisat zeichnet sich durch gute Rieselfähigkeit, lange Lagerbarkeit ohne zu verbacken, geringe Restfeuchte vor dem Trocknungsschritt sowie durch eine im Vergleich zu herkömmlichen Kristallisaten deutlich erhöhte mittlere Kristallgröße aus.

Ein weiterer Vorteil der Erfindung liegt darin, daß bei Verwendung eines hochmolekularen anionischen Polyelektrolyten, wie er im Rahmen der vorliegenden Erfindung definiert ist, im Gegensatz zur Verwendung von niedermolekularen Verbindungen als Kristallisationshilfsmittel bereits äußerst geringe Konzentrationen, beispielsweise wenige ppm bezogen auf die gesamte Kristallisationslösung, ausreichen, um den gewünschten Effekt eines rieselfähigen und lagerfähigen Kristallisats, das eine im Vergleich zu herkömmlichen Methoden erhöhte mittlere Kristallgröße bei enger Kristallgrößenverteilung aufweist, und damit arm an Feingutanteilen ist, zu erzielen.

Bei Zugabe solch geringer Mengen an Kristallisationshilfsmittel, wie sie im Rahmen der Erfindung vorgesehen sind, bleibt im Gegensatz zu herkömmlichen monomeren Additiven höchstens eine nur gerade noch meßbare Menge an Kristallisationshilfsmittel im Kristallisat, welche keine signifikante, die Weiterverarbeitbarkeit negativ beeinflussende Verunreinigung der Adipinsäure verursacht. In der Regel liegt der Anteil an Kristallisationshilfsmittel im Kristallisat bei der Anwendung des erfindungsgemäßen Verfahrens unterhalb 20 ppm, vorzugsweise unterhalb von 10 ppm und besonders bevorzugt unterhalb von 5 ppm.

Die niedrigen Konzentrationen erlauben es zudem, Störungen, wie beispielsweise durch Tenside hervorgerufene Schaumbildung während mit der Kristallisation von Adipinsäure verbundenen Verfahrensschritten sowie ggf. in späteren Verarbeitungsschritten zu vermeiden. Durch die Zugabe des Kristallisationshilfsmittels wird eine Erhöhung der mittleren Kristallgröße um bis zu 50% und ein nach visueller Beurteilung störungsärmeres Kristallgitter erzeugt. Diese Effekte führen zu einer größeren Härte und geringeren Abriebempfindlichkeit (Verringerung des Feinanteils in nachgeschalteten Verfahrensschritten) des Kristallisats. Weiterhin wird eine verbesserte Abtrennbarkeit von Wasser aus dem frischen Kristallisat und damit eine geringere Restfeuchte des Kristallisats vor dem Trocknungsschritt erreicht (erleichterte Trocknung).

Als Dicarbonsäure zum Einsatz im Rahmen des erfindungsgemäßen Verfahrens sind alle organischen Dicarbonsäuren geeignet. Insbesondere sind dies Oxalsäure, Malonsäure, Bernsteinsäure, Glutarsäure, Adipinsäure, Pimelinsäure, Maleinsäure, Fumarsäure sowie weitere höhere, gesättigte oder ungesättigte, verzweigte oder unverzweigte Dicarbonsäuren. Die Dicarbonsäuren können gegebenenfalls noch weitere funktionelle Gruppen wie beispielsweise Hydroxygruppen oder Substituenten, wie beispielsweise Halogenatome, aufweisen.

Der Einsatz von Adipinsäure stellt bei der Durchführung des erfindungsgemäßen Verfahrens eine bevorzugte Ausführungsform dar.

Beim erfindungsgemäßen Verfahren zur Herstellung der Dicarbonsäurekristallisate wird zu einer Lösung, die mindestens die zu kristallisierende Dicarbonsäure enthält, mindestens ein anionischer Polyelektrolyt mit einer Molmasse von wenigstens 2.000 gegeben. Vorteilhafterweise weist der Polyelektrolyt eine Molmasse von 20.000 bis 2.000.000 auf, wobei eine Molmasse von 100.000 bis 500.000 im Sinne der Erfindung bevorzugt ist. In einer vorteilhaften Ausführungsform der Erfindung wird ein Polyelektrolyt mit einer Molmasse von 200.000 bis 300.000 eingesetzt.

Als anionischer Polyelektrolyt ist grundsätzlich jedes Makromolekül geeignet, das eine zur Erzielung des erfindungsgemäßen Effekts ausreichende Anzahl anionischer Gruppen im Molekül aufweist. In der Regel handelt es sich dabei um anionische Gruppen, die entweder endständig am Molekül und/oder als Seitengruppe am oligomeren oder polymeren Rückgrat des anionischen Polyelektrolyten angebracht sind.

Unter dem Begriff *"anionisch"* oder "*anionische Gruppen*" werden im Sinne der vorliegenden Erfindung sowohl funktionelle Gruppen verstanden, die erst durch Zugabe einer basischen Verbindung, in der Regel unter Abspaltung eines Protons, in die anionische Form übergehen als auch solche funktionellen Gruppen, die schon in der anionischen Form mit einem geeigneten Gegenion vorliegen.

Als Gegenionen sind beispielsweise Metallkationen geeignet. Insbesondere sind dies die Kationen der Alkalimetalle, wie beispielsweise Lithium, Natrium oder Kalium. Ebenfalls als Gegenionen geeignet sind die quarternären Ammoniumionen, wie sie beispielsweise aus Aminoverbindungen durch Protonierung mit Säuren erhältlich sind.

Vorzugsweise werden jedoch im Sinne der vorliegenden Erfindung sogenannte anionische Polyelektrolyte eingesetzt, deren anionische Gruppen in der sauren, d.h. in der nicht neutralisierten Form vorliegen.

Es ist im Rahmen der vorliegenden Erfindung bevorzugt, anionische Polyelektrolyte einzusetzen, die in wäßriger Lösung, vorzugsweise in Wasser selbst wenigstens wasserdispergierbar sind, da im Rahmen der vorliegenden Erfindung bevorzugt aus wäßriger Lösung kristallisiert wird. Vorzugsweise ist der anionische Polyelektrolyt jedoch wasserlöslich, wobei unter Wasserlöslichkeit die Ausbildung molekulardisperser Lösungen der anionischen Polyelektrolyte verstanden wird. Da die Wasserlöslichkeit des anionischen Polyelektrolyten wenigstens weitgehend durch seine anionischen Gruppen bestimmt wird, ist es damit bevorzugt solche anionischen Polyelektrolyte einzusetzen, die eine zur Erzeugung von Wasserlöslichkeit ausreichende Anzahl an anionischen Gruppen aufweisen.

Es ist jedoch ebenso denkbar anionische Polyelektrolyte einzusetzen, deren Zahl an anionischen Gruppen zur Erzeugung von Wasserlöslichkeit nicht ausreichend ist. Solche anionischen Polyelektrolyte weisen dann jedoch zur Herstellung von Wasserlöslichkeit andere hydrophile Einheiten im Molekül auf, beispielsweise Polyethereinheiten.

Im Rahmen des erfindungsgemäßen Verfahrens wird der mindestens die zu kristallisierende Dicarbonsäure enthaltenden Lösung mindestens ein anionischer Polyelektrolyt zugesetzt. Es können im Sinne der Erfindung jedoch auch Gemische aus zwei oder mehr verschiedenen anionischen Polyelektrolyten als Kristallisationshilfsmittel eingesetzt werden.

Hierbei können auch Gemische aus anionischen Polyelektrolyten mit unterschiedlichen Molmassen zum Einsatz kommen.

Der Polyelektrolyt wird in einer Menge von 0,01 ppm bis 200 ppm, bezogen auf die zu kristallisierende Lösung, zugegeben. Zugaben höherer Mengen zur Erzielung des erfindungsgemäßen Effekts sind ebenfalls möglich, bringen jedoch in der Regel keine Verbesserung des Kristallisats mit sich.

Die beim erfindungsgemäßen Verfahren eingesetzte Lösung enthält damit neben der zu kristallisierenden Dicarbonsäure mindestens einen anionischen Polyelektrolyten in einer Menge von 0,01 bis 200 ppm, vorzugsweise jedoch in einer Menge von 0,05 bis 200 ppm oder 0,1 bis 150 ppm. Besonders bevorzugt wird der Polyelektrolyt in einer Menge von 1 bis 80 ppm eingesetzt. Die Mengenangaben beziehen sich dabei jeweils auf die gesamte zu kristallisierende Lösung.

Angesichts der im Rahmen der vorliegenden Erfindung geringen zuzugebenden Menge an anionischem Polyelektrolyten und dem großen Überschuß an auszukristallisierender Dicarbonsäure kann unter Inkaufnahme geringster Verunreinigungen mit Spuren der zur Neutralisation des anionischen Polyelektrolyten benutzten Base auch ein anionischer Polyelektrolyt mit neutralisierten anionischen Gruppen eingesetzt werden. Die Verwendung neutralisierter, anionischer Polyelektrolyte ist jedoch nicht bevorzugt.

Der anionische Polyelektrolyt kann als funktionelle Gruppe beispielsweise Carbonsäuregruppen, Sulfonsäuregruppen oder Phosphonsäuregruppen oder Gemische aus zwei oder mehr davon tragen. Vorzugsweise trägt der anionische Polyelektrolyt jedoch Carbonsäure- oder Sulfonsäuregruppen, wobei Carbonsäuregruppen im Sinne der Erfindung bevorzugt sind.

Als mindestens ein anionischer Polyelektrolyt eignet sich beispielsweise ein Polymerisat, hergestellt aus Monomeren der Acrylsäure, Methacrylsäure, Maleinsäure, Fumarsäure, Itaconsäure oder Mesaconsäure, oder ein Copolymerisat, hergestellt aus zwei oder mehr dieser Monomeren, oder ein Copolymerisat, hergestellt aus mindestens einem dieser Monomeren und mindestens einem weiteren Monomeren, das frei von Carboxylgruppen ist, oder ein Gemisch aus zwei oder mehr dieser Polymerisate oder Copolymerisate.

Als von Carboxylgruppen freie Monomere sind beispielsweise Vinylacetat, Acrylamid, Isobuten oder andere der Polymerisation zugängliche Olefine geeignet. Der Polyelektrolyt enthält die von Carboxylgruppen freien Monomeren in der Regel in einem Anteil von bis zu 40 Gew.-%, vorzugsweise in einem Anteil von lediglich bis zu 30 Gew.-%. Gegebenenfalls kann jedoch auch ein geringerer Anteil, wie beispielsweise 20 Gew.-%, 15 Gew.-%, 10 Gew.-% oder noch weniger, beispielsweise lediglich 5 Gew.-% oder darunter, vorteilhaft sein.

Zur Durchführung der erfindungsgemäßen Verfahrens ist es vorteilhaft, wenn der anionische Polyelektrolyt in dem zum Lösen der Dicarbonsäure benutzten Lösemittel selbst ebenfalls löslich ist. Aufgrund der geringen zuzugebenden Menge ist es jedoch nicht erforderlich, daß die Löslichkeit derjenigen der Dicarbonsäure entspricht oder sie sogar übersteigt. Es können gegebenenfalls auch anionische Polyelektrolyte mit einer geringeren Löslichkeit zugegeben werden.

In einer bevorzugten Ausführungsform der Erfindung wird als anionischer Polyelektrolyt Polyacrylsäure mit eines Molmasse von etwa 250.000 eingesetzt.

Die Kristallisation der Dicarbonsäure erfolgt beim erfindungsgemäßen Verfahren aus einem Lösemittel oder einem Lösemittelgemisch. Im Rahmen der Erfindung werden auch Lösemittelgemische als "Lösemittel" bezeichnet, wenn nichts ausdrücklich etwas anderes angegeben wird.

Grundsätzlich sind für das erfindungsgemäße Verfahren alle Lösemittel geeignet, in denen die zu kristallisierende Dicarbonsäure und das Kristallisationshilfsmittel eine ausreichende Löslichkeit aufweisen. In der Regel ist es vorteilhaft, wenn ein Lösemittel gewählt wird, in dem die Dicarbonsäure bei erhöhter Temperatur eine sehr gute Löslichkeit aufweist, die Löslichkeit bei Verringerung der Temperatur jedoch abnimmt, vorzugsweise stark abnimmt. Hierzu können sowohl organische Lösemittel als auch Wasser oder Gemische von Wasser und einem oder mehreren organischen Lösemitteln eingesetzt werden. In der Regel sollte jedoch bei der Verwendung von wäßrigen Lösemitteln eine Bildung von mehreren Lösemittelphasen im Lösemittelgemisch vermieden werden.

Wenn daher ein Gemisch aus Wasser und einem organischen Lösemittel oder ein Gemisch aus Wasser und einem Gemisch aus mehreren organischen Lösemitteln im erfindungsgemäßen Verfahren eingesetzt wird, so sollte das organische Lösemittel oder das Gemisch aus mehreren organischen Lösemitteln wenigstens begrenzt wasserlöslich sein und dem Wasser in einer Menge zugegeben werden, bei der keine Phasentrennung auftritt. Als wassermischbare, organische Lösemittel kommen beispielsweise Ketone, wie Aceton oder Methylethylketon, oder Alkohole, wie Methanol oder Ethanol, in frage.

Es ist jedoch im Sinne der Erfindung bevorzugt, als Lösemittel Wasser einzusetzen.

Eine Kristallisation erfolgt in der Regel dann, wenn eine übersättigte Lösung der Dicarbonsäure vorliegt. Zum Auslösen der Kristallisation bestehen verschiedene Möglichkeiten. Zum einen ist es möglich, eine Übersättigung der Lösung dadurch zu erzeugen, daß die Lösemittelmenge, beispielsweise durch kontinuierliches Abdampfen des Lösemittels bei Normaldruck oder unter verringertem Druck, erniedrigt wird.

Eine weitere Möglichkeit besteht darin, die Dicarbonsäure im Lösemittel bei erhöhter Temperatur zu lösen, und durch Abkühlen des Lösemittels auf eine darunterliegende Temperatur eine Übersättigung zu erzeugen, die schließlich zur Kristallisation führt.

Es ist ebenfalls möglich, beide Verfahren gleichzeitig anzuwenden. Das heißt, daß die Lösung auf eine Temperatur abgekühlt wird, die unterhalb derjenigen liegt, bei der die Dicarbonsäure gelöst wurde, wobei die Temperatur jedoch noch so hoch ist, daß das Lösemittel, ggf. unter Anlegen eines Vakuums, noch verdampft.

Prinzipiell kann die Kristallisation zwar bei beliebigen Temperaturen durchgeführt werden, da sie hauptsächlich aufgrund der Löslichkeitsdifferenz im Lösemittel bei unterschiedlichen Temperaturen bzw. aufgrund der Abnahme der Lösemittelmenge durch Verdampfen erfolgt. Vorteilhafterweise wird jedoch aus praktischen Erwägungen eine Temperatur von zwischen 0°C und 100°C gewählt werden.

Das erfindungsgemäße Verfahren kann mit beliebigen Lösungen beliebiger organischer Dicarbonsäuren in den oben beschriebenen Lösemitteln, vorzugsweise in Wasser, durchgeführt werden. Zur Herstellung dieser Lösungen wird in der Regel eine Dicarbonsäure bei erhöhter Temperatur im Lösemittel gelöst. Es wird hierbei üblicherweise soviel Dicarbonsäure zugesetzt, wie zum Ausbilden einer gesättigten Lösung notwendig ist. Gewünschtenfalls kann jedoch auch mit Lösungen gearbeitet werden, die unterhalb dieser Sättigungsgrenze liegen.

Wird als Dicarbonsäure beispielsweise Adipinsäure eingesetzt, so empfiehlt es sich, die Kristallisation ausgehend von einer 30 bis 60%-igen Lösung von Adipinsäure in Wasser (70-95°C) durchzuführen.

Die Kristallisation wird durch Abkühlen der Lösung oder durch Abdampfen des Lösemittels, ggf. unter Anlegen eines Vakuums, oder durch eine Kombination aus beiden Methoden eingeleitet. Vor oder während der Kristallisation kann nun ein Polyelektrolyt mit einer Molmasse von wenigstens 2.000. zugesetzt werden. Vorzugsweise erfolgt die Zugabe des Polyelektrolyten vor der Kristallisation.

Die Kristallisation wird im Rahmen eines kontinuierlichen oder eines diskontinuierlichen Verfahrens durch Verdampfen des Lösemittels, Kühlen des Lösemittels oder einer Kombination aus beiden Verfahren eingeleitet und durchgeführt, üblicherweise bis zu einer Suspensionsdichte von 30 bis 40 Gew.-% Feststoff. Das dabei gewonnene Kristallisat kann auf Zentrifugen sowie auch auf anderen Abtrennorganen, beispielsweise Nutschen, abgetrennt und mit beliebigen Verfahren getrocknet werden. Es weist ohne weitere Behandlung die eingangs bezeichneten Vorzüge auf.

Das erfindungsgemäße Verfahren kann in allen zu diesem Zweck benutzbaren Vorrichtungen durchgeführt werden.

Die organische Dicarbonsäure ist im nach dem erfindungsgemäßen Verfahren erhaltenen Dicarbonsäurekristallisat in der Regel in einer Menge von wenigstens 99,5 Gew.-% enthalten. Wenn auf besonders reine Dicarbonsäurekristallisate Wert gelegt wird, liegt der Gehalt an organischer Dicarbonsäure entsprechend höher. So können die erfindungsgemäß erhaltenen Dicarbonsäurekristallisate die organische Dicarbonsäure in einer Menge von wenigstens 99,5, 99,9, 99,95 oder sogar 99,99 Gew.-% oder mehr enthalten (bezogen auf Trockensubstanz).

Neben der organischen Dicarbonsäure und dem anionischen Polyelektrolyten kann das erfindungsgemäß erhaltene Dicarbonsäurekristallisat in geringen Mengen noch weitere Stoffe, in der Regel Verunreinigungen, enthalten. Hierbei ergibt der Anteil an Dicarbonsäure zusammen mit dem Anteil an anionischen Polyelektrolyten sowie ggf. vorhandenen weiteren Inhaltsstoffen 100% des Kristallisats.

Vorzugsweise enthält das erfindungsgemäß erhaltene Kristallisat als Dicarbonsäure Adipinsäure. Als weitere Inhaltsstoffe können hier beispielsweise die üblichen, bei der Herstellung von Adipinsäure durch Oxidation eines Cyclohexanon/Cyclohexanol-Gemisches anfallenden Verunreinigungen, wie beispielsweise Maleinsäure, Glutarsäure, Bernsteinsäure, Capronsäure, Salpetersäure und Lösemittelreste, wie beispielsweise Wasser, vorliegen.

Die weiteren Inhaltsstoffe oder Verunreinigungen sind dabei in der Regel in einer Menge von 1000 ppm, bevorzugt weniger als 200 ppm und besonders bevorzugt weniger als 10 ppm (bezogen auf den jeweiligen Inhaltsstoff oder die jeweilige Verunreinigung) im Kristallisat vorhanden.

Ebenfalls Gegenstand der Erfindung ist die Verwendung eines anionischen Polyelektrolyten mit einer Molmasse von mindestens 2.000, zur Herstellung von Dicarbonsäurekristallisaten.

Die folgenden Beispiele dienen der näheren Erläuterung des erfindungsgemäßen Verfahrens.

### Beispiele

### Beispiel 1:

In einem kontinuierlich betriebenen 1 1 Laborkristallisator wird eine 35 Gew.-%-ige Lösung von Adipinsäure in Wasser mit 80°C vorgelegt und mit 15 ppm einer Polyacrylsäure (bezogen auf die gesamte Lösung) des Molmasse 250.000 versetzt. Nach Reduzierung des Drucks auf 200 mbar wird durch Abdampfen von Wasser und Kühlung auf 60°C die Feststoffbildung eingeleitet. Unter Zuführen frischen Feeds der gleichen Zusammensetzung sowie periodischem Abziehen kleiner Suspensionsmengen wird die Lösung über 8 Stunden bis zur Einstellung eines stationären Zustandes kontinuierlich kristallisiert. Am Ende des Versuches wird die im Kristallisator enthaltene Suspension durch dreiminütiges Schleudern auf einer Siebbecherzentrifuge bei 600 g abgetrennt. Es werden ca. 200 g Kristalle mit einer Restfeuchte von 3,2% und einer mittleren Kristallgröße von 571 µm gewonnen. Nach halbstündigem Trocknen im Wasserstrahlvakuum bei 60°C werden die Kristallite in einem geschlossenen Gefäß gelagert. Nach vier Wochen ist das aus isometrischen Partikeln bestehende Kristallisat frei rieselfähig.

Im Unterschied zu bekannten Kristallformen der Adipinsäure sind die nach dem Beispiel erhältlichen Kristalle bei größerem mittleren Durchmesser deutlich kompakter, haben damit eine eine geringere Oberfläche als nach herkömmlichen Verfahren erhältliche Kristalle mit vergleichbarem mittlerem Durchmesser und weisen im wesentlichen hydrophobe Flächen als nach außen gerichtete Flächen auf.

### Vergleichsbeispiel:

Der gleiche Versuch ohne Kristallisationshilfsmittel liefert ein Kristallisat in Form dünner Plättchen mit nur 432 µm mittlerer Kristallgröße und einer anfänglichen Restfeuchte von 4,7%. Es ist nach nur 24-stündiger Lagerung im geschlossenen Gefäß mäßig, nach einigen Wochen extrem verbacken.

## Patentansprüche

1. Verfahren zur Herstellung von Dicarbonsäurekristallisaten aus einer mindestens eine organische Dicarbonsäure enthaltenden Lösung, **dadurch gekennzeichnet, daß** der Lösung vor oder während der Kristallisation mindestens ein anionischer Polyelektrolyt mit einer Molmasse von wenigstens 2000, in einer Menge von 0,01 bis 200 ppm zugegeben wird.

2. Verfahren nach Anspruch 1, **dadurch gekennzeichnet, daß** als mindestens ein anionischer Polyelektrolyt ein Polymerisat, hergestellt aus Monomeren der Acrylsäure, Methacrylsäure, Maleinsäure, Fumarsäure, Itaconsäure oder Mesaconsäure, oder ein Copolymerisat, hergestellt aus zwei oder mehr dieser Monomeren, oder ein Copolymerisat, hergestellt aus mindestens einem dieser Monomeren und mindestens einem weiteren Monomeren, das frei von Carboxylgruppen ist, oder ein Gemisch aus zwei oder mehr dieser Polymerisate oder Copolymerisate zugegeben wird.

3. Verfahren nach einem der vorstehenden Ansprüche, **dadurch gekennzeichnet, daß** der mindestens eine anionische Polyelektrolyt eine Molmasse von 20.000 bis 2.000.000, vorzugsweise 100.000 bis 500.000, aufweist.

4. Verfahren nach einem der vorstehenden Ansprüche, **dadurch gekennzeichnet, daß** als mindestens ein anionischer Polyelektrolyt Polyacrylsäure mit einer Molmasse von 250.000 eingesetzt wird.

5. Verfahren nach einem der vorstehenden Ansprüche, **dadurch gekennzeichnet, daß** der mindestens eine anionische Polyelektrolyt in einer Menge von 0,1 bis 150 ppm eingesetzt wird.

6. Verfahren nach einem der vorstehenden Ansprüche, **dadurch gekennzeichnet, daß** als Dicarbonsäure Adipinsäure eingesetzt wird.

7. Verwendung mindestens eines anionischen Polyelektrolyten mit einer Molmasse von mindestens 2000 bei der Herstellung von Dicarbonsäurekristallisaten.

## Claims

1. A process for preparing dicarboxylic acid crystals from a solution containing at least one organic dicarboxylic acid, wherein at least one anionic polyelectrolyte having a molecular weight of at least 2000 is added to the solution before or during the crystallization in an amount of from 0.01 to 200 ppm.

2. A process as claimed in claim 1, wherein a polymer prepared from acrylic acid, methacrylic acid, maleic acid, fumaric acid, itaconic acid or mesaconic acid monomers, or a copolymer prepared from two or more of these monomers, or a copolymer prepared from at least one of these monomers and at least one other monomer which is free of carboxyl groups, or a mixture of two or more of these polymers or copolymers, is added as at least one anionic polyelectrolyte.

3. A process as claimed in either of the preceding claims, wherein the at least one anionic polyelectrolyte has a molecular weight of from 20,000 to 2,000,000, preferably 100,000 to 500,000.

4. A process as claimed in any of the preceding claims, wherein polyacrylic acid having a molecular weight of 250,000 is employed as at least one anionic polyelectrolyte.

5. A process as claimed in any of the preceding claims, wherein the at least one anionic polyelectrolyte is employed in an amount of from 0.1 to 150 ppm.

6. A process as claimed in any of the preceding claims, wherein adipic acid is employed as dicarboxylic acid.

7. The use of at least one anionic polyelectrolyte having a molecular weight of at least 2000 for preparing dicarboxylic acid crystals.

## Revendications

1. Procédé de préparation de cristaux d'acides dicarboxyliques à partir d'une solution contenant au moins un acide dicarboxylique organique, **caractérisé en ce qu'**on ajoute à la solution, avant ou après la cristallisation, au moins un polyélectrolyte anionique ayant une masse moléculaire d'au moins 2000, en une quantité de 0,01 à 200 ppm.

2. Procédé selon la revendication 1, **caractérisé en ce qu'**on ajoute, en tant qu'au moins un polyélectrolyte anionique, un polymère préparé à partir de monomères de l'acide acrylique, de l'acide méthacrylique, de l'acide maléique, de l'acide fumarique, de l'acide itaconique ou de l'acide mésaconique, ou encore un copolymère préparé à partir d'au moins deux de ces monomères, ou encore un copolymère préparé à partir d'au moins l'un de ses monomères et d'au moins un autre monomère, qui est exempt de groupes carboxyle, ou encore un mélange d'au moins deux de ces polymères ou copolymères.

3. Procédé selon l'une des revendications précédentes, **caractérisé en ce que** le ou les polyélectrolytes anioniques ont une masse moléculaire de 20 000 à 2 000 000 et de préférence de 100 000 à 500 000.

4. Procédé selon l'une des revendications précédentes, **caractérisé en ce qu'**on utilise, en tant qu'au moins un polyélectrolyte anionique, un poly(acide acrylique) ayant une masse moléculaire de 250 000.

5. Procédé selon l'une des revendications précédentes, **caractérisé en ce que** le ou les polyélectrolytes anioniques sont utilisés en une quantité de 0,1 à 150 ppm.

6. Procédé selon l'une des revendications précédentes, **caractérisé en ce que** l'acide dicarboxylique utilisé est l'acide adipique.

7. Utilisation d'au moins un polyélectrolyte anionique ayant une masse moléculaire d'au moins 2000, lors de la préparation de cristaux d'acides dicarboxyliques.
